(19) Europäisches Patentamt
European Patent Office
Office européen des brevets

(11) **EP 2 444 057 B1**

(12) **EUROPÄISCHE PATENTSCHRIFT**

(45) Veröffentlichungstag und Bekanntmachung des Hinweises auf die Patenterteilung:
**30.05.2018 Patentblatt 2018/22**

(51) Int Cl.:
*A61K 8/19* (2006.01)    *A61K 8/34* (2006.01)
*A61K 8/365* (2006.01)    *A61K 8/41* (2006.01)
*A61Q 5/00* (2006.01)    *A61Q 5/12* (2006.01)

(21) Anmeldenummer: **11159118.6**

(22) Anmeldetag: **22.03.2011**

(54) **Haarbehandlungsmittel mit einem hohen Anteil an gebundenem Wasser**

Hair treatment agent with a high proportion of bound water

Produit de traitement capillaire ayant une teneur en eau liée élevée

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.07.2010 DE 102010026747**

(43) Veröffentlichungstag der Anmeldung:
**25.04.2012 Patentblatt 2012/17**

(73) Patentinhaber: **Beiersdorf AG**
**20253 Hamburg (DE)**

(72) Erfinder:
• **Kudla, Petra**
**22525, Hamburg (DE)**

• **Sokolowski, Tobias**
**20257, Hamburg (DE)**
• **Saladin, Sandra**
**20144, Hamburg (DE)**

(56) Entgegenhaltungen:
**EP-A2- 2 138 154    WO-A1-03/094867**
**WO-A1-2008/074617    WO-A2-99/34768**

• **DATABASE GNPD [Online] MINTEL; 31. Mai 2010 (2010-05-31), "1 Minute Mask", XP002724200, Database accession no. 1321468**

**Beschreibung**

Die Erfindung betrifft neuartige Haarbehandlungsmittel mit einem hohen Anteil an gebundenem Wasser.

Haarbehandlungsmittel werden zur Pflege nach der Reinigung der Haare - einer Form keratinischer Fasern - angewendet. Spülungen und Kuren werden normalerweise auf Basis von kationischen Tensiden wie beispielsweise Cetyltrimethylammoniumchlorid und Fettalkoholen wie Cetyl- und/oder Stearylalkohol formuliert. Insbesondere eine hohe Cremigkeit und Pflegeleistung während der Anwendung ist für den Verbraucher besonders wichtig.

Alle Mengenangaben sind- sofern nicht anders angegeben - Massenprozente bezogen auf die Gesamtmasse der Zubereitung.

Tenside im Sinne der vorliegenden Schrift sind oberflächenaktive Verbindungen, also sowohl Reinigungstenside, als auch Emulgatoren und oberflächenaktive Polymere.

Die Wasserbindung einer Formulierung lässt sich laut Junginger (Pharm. Ind. 46 (1984) 758) in einen freie Wasserphase (Bulkwasser) und eine interlamellar gebundene Wasserphase aufteilen. Die Art der Wasserbindung lässt sich über den Diffusionskoeffizienten der Wassermoleküle in der Formulierung bestimmen. Dieser kann mit Hilfe von NMR-Diffusionsexperimenten nach der Methode von Stejskal und Tanner (J. Chem. Phys. 42 (1965), 288) gemessen werden. Bei den beschriebenen Formulierungen bietet es sich an, Experimente nach dem Verfahren des stimulierten Echos (Tanner, J. Chem. Phys. 52 (1970), 2523) durchzuführen mit Diffusionszeiten (DELTA) zwischen 50 ms und 2 s, Gradientendauern (delta) zwischen 1 und 5 ms sowie Gradientenstärken (G) von 0 bis 100 G/cm. Aus dem Verlauf der normierten Signalintensitäten (I/I0) bei verschiedenen Gradientenstärken kann der Diffusionskoeffizient (D) über folgende Gleichung bestimmt werden:

$$I/I0 = exp(-gamma2*G2*delta2*D*( DELTA-delta/3 ))$$

In heterogenen Systemen, wie den hier beschriebenen Zubereitungen, lassen sich für die verschiedenen Wasseranteile unterschiedliche Diffusionskoeffizienten bestimmen. In reinem Wasser bei 25°C beträgt der Diffusionskoeffizient von Wasser D = $2,3\times10^{-9}$ m$^2$/s. Wasser mit einem signifikant niedrigerem Diffusionskoeffizienten von $< 8\times10^{-11}$ m$^2$/s wird gemäß dieser Erfindung als gebundenes Wasser betrachtet.

Die Schrift WO 2009158441 A1 offenbart Reinigungszubereitungen mit einer Mischung aus Stearamidopropyl Dimethylamine und L-Glutaminsäure.

WO 2009158442 offenbart Reinigungszubereitungen mit einer Mischung aus Behenamidopropyl Dimethylamine und L-Glutaminsäure.

Die Schrift EP 1 254 653 B1 offenbart Reinigungszubereitungen für keratinische Fasern mit Esterquats und Fettsäureamidoaminen.

EP 1131041 B1 offenbart Zubereitungen mit einer Mischung aus Fettalkoholen, Monoalkylquats und Glykolen.

US 7601339 B2 offenbart Reinigungszubereitungen mit einer Mischung aus langkettigen Amidoaminen und Stearylalkohol.

WO 2006137003 A2 offenbart Reinigungszubereitungen mit einer Mischung aus kationischen Tensiden, Alkylsulfaten und Fettalkoholen. Diese bilden eine lamellare Gelmatrix.

EP 2138154 A2 offenbart eine Haarspülung mit Amphotensid und besonderer Lagerstabilität.

All dies konnte die Mängel des Standes der Technik nicht beseitigen. Dem Stand der Technik fehlte es an Zubereitungen, die eine besondere Reichhaltigkeit aufweisen. Reichhaltigkeit bedeutet, dass der Anwender beim Einmassieren des Produktes in das Haar das Produkt als besonders cremig wahrnimmt, sich das Produkt also nicht dünn oder wässrig anfühlt. Dabei sollen gleichzeitig die haarkonditionierenden Eigenschaften gut sein.

Überraschend und für den Fachmann nicht vorhersehbar hat sich nun herausgestellt, dass eine kosmetische Zubereitung zur Behandlung keratinischer Fasern enthaltend kationische Tenside, wobei die kationischen Tenside Stearamidopropyl Dimethylamine, Behenamidopropyl Dimethylamine und/oder Palmitamidopropyltrimonium Chloride sind, einen oder mehrere Fettalkohole mit einem Schmelzpunkt oberhalb 35°C und Milchsäure dadurch gekennzeichnet, dass der Anteil an interlamellar gebundenem Wasser wenigstens 50 %; gemessen mit $^1$H-NMR Diffusionsmessungen bei 25°C, beträgt und dadurch gekennzeichnet, dass das Stoffmengenverhältnis zwischen kationischen Tensiden zu der Summe der Stoffmengen an allen Tensiden und Fettverbindungen 10 bis 20% beträgt, den Mängeln des Standes der Technik abhilft. Durch solche Formulierungen wird eine sehr reichhaltige, cremige Spülungstextur erreicht. Zugleich sind Verteilbarkeit des Produktes im Haar, das Gleitvermögen eines Kammes durch das Haar bei Anwendung des Produktes, die Trockenkämmbarkeit sehr gut. Dabei ist es bevorzugt, wenn die Fettalkohole C14, C16, C18 Alkohole sind, ganz besonders bevorzugt eine Mischung aus Cetyl- und Stearylalkohol, da diese zu einem höheren Anteil an gebundenem Wasser führen und die reichhaltige Sensorik der Zubereitung weiter verbessert wird. Das Stoffmengenverhältnis zwischen kationischen Tensiden zu der Summe der Stoffmengen an allen Tensiden und Fettverbindungen von 10 bis 20% bewirkt eine höhere interlamellare Einbindung des Wassers und damit eine weitere Steigerung der reichhaltigen Sensorik. Bevorzugt ist es, wenn der Anteil an interlamellar gebundenem Wasser wenigstens 60% beträgt. Bei all dem ist es besonders bevorzugt, wenn Fettalkohole in Mengen von 5,0 bis 8,5 Gew.-%, vorzugsweise 6,5 bis 8,0 Gew.-%, bezogen auf die gesamte Zusammensetzung, enthalten sind, wodurch die reichhaltige Sensorik der Zubereitung noch weiter verbessert wird. Die Verwendung von Stearamidopropyl Dimethylamine, Behenamidopropyl Dimethylamine und/oder Palmitamidopropyltrimonium Chloride als kationisches Tensid verbessert die reichhaltige Sensorik der Zubereitung weiter. Die Erfindung umfasst auch die Verwendung von erfindungsgemäßen Zubereitungen zur Verbesserung der Pflege und Kämmbarkeit der Haare.

Gemäß der Erfindung sind tertiäre Amine mit einer zusätzlichen Amidfunktion, bekannt als Amidoamine, besonders bevorzugt als kationische Tenside in einem sauren Medium. Besonders bevorzugt ist die Verwendung von einem Amidoamin, bekannt als Stearamidopropyl Dimethylamine bekannt als Tego Amid S 18 von Evonik, sowie auch Behenamidopropyl Dimethylamine, bekannt als Amidet APA-22 von Kao oder Palmitamidopropyltrimonium Chloride, bekannt als Varisoft PATC von Evonik. Diese Amide werden in den erfindungsgemäßen Zusammensetzungen in einer Menge von 1,0 bis maximal 3,5 Gew.-%, bevorzugt 1,0 - 2,5 Gew.-% eingesetzt.

Erfindungsgemäße Spülungen können auch Monoalkyltrimethylammoniumsalze mit einer Kettenlänge des Alkylrestes von 16 bis 24 Kohlenstoffatomen enthalten. Diese Verbindungen weisen folgende Struktur auf:

$$[RR'R''R'''N]^+ X^-$$

wobei R, RI und RII für jeweils eine Methylgruppe stehen und RIII für einen gesättigten, verzweigten oder unverzweigten Alkylrest mit einer Kettenlänge von 16 bis 24 Kohlenstoffatomen. X- steht für ein Anion, ausgewählt aus den physiologisch verträglichen Anionen.

Beispielhaft für das Anion seien die Halogenide Fluorid, Chlorid oder Bromid sowie Sulfat (Methosulfat) der allgemeinen Formel $RSO_3^-$ worin R die Bedeutung von gesättigtem oder ungesättigtem Alkylresten mit 1 bis 4 Kohlenstoffatomen hat, oder anionische Reste organischer Säuren wie Maleat, Fumarat, Oxalat, Tartrat, Citrat, Lactat oder Acetat, genannt.

Beispiele für Verbindungen dieser Formel sind Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat. Bevorzugt sind Cetyltrimethylammonium- und Behenyltrimethylammoniumsalze. Besonders bevorzugt sind letztere in Form der Methosulfate, Chloride und Bromide. Am bevorzugtesten wird Cetyltrimethylammoniumchlorid und höchst bevorzugt ist Behenyl-trimethylammoniumchlorid. Diese können in Kombination mit Amidoamin oder alleine eingesetzt werden. Besonders Vorteilhaft ist die Kombination von einem Amidoamin mit einer Monoalkyltrimethylammonium-Verbindung in einem Verhältnis von 1:1.

Die Verbindungen der angegebenen Formel werden in den erfindungsgemäßen Zusammensetzungen in einer Menge von 0,01 bis 5,0 Gew.-% verwendet. Bevorzugt werden 0,1 bis 5 Gew.-% verwendet. Besonders bevorzugt sind Mengen von 0,5 bis 4,0 Gew.-%. Die Mengenangaben beziehen sich jeweils auf die gesamte Zusammensetzung.

Gemäß der Erfindung wird es bevorzugt, wenn ein Silikonöl vorhanden ist. Wenn als solches ein Polydimethylsiloxan (Dimethicone) in Form einer Emulsion verwendet wird, beträgt die Tröpfchengröße der emulgierten Teilchen erfindungsgemäß 0,01 $\mu$m bis 10000 $\mu$m, bevorzugt 0,01 bis 100 $\mu$m, ganz besonders bevorzugt größere Partikel von 0,3 bis 30 $\mu$m. Die Teilchengröße wird dabei nach der Methode der Lichtstreuung bestimmt. Bevorzugt sind Viskositäten der Silikonemulsionen von bis 5000 -12000 mPa s (Haake Viscotester VT 550 mit dem Messsystem NV bei 25°C). Dimethicone kann alleine in der Emulsion sowie auch als Mischung mit einem weiteren Silikon vorhanden sein. Beispiele sind als Handelsnamen Dow Corning 5-7139, Dow Corning 2-1352 und Dow Corning 2-1491 Emulsionen bekannt.

Dimethicone kann auch als Fluid vorhanden sein. Bekannte Handelsnamen sind Xiameter® PMX-200 Silicone Fluid Serie von Dow Corning mit kinematischen Viskositäten von 50 cSt bis maximal 60000 cSt. Weitere Handelsnamen für Dimethicone sind unter Wacker Belsil DM 3 Grades, BRB Silicone Oil DM Grades, SF96 Serie von Momentive Performance Materials und DM Fluid Serien von Shin-Etsu Chemicals Co. bekannt. Ein weiteres bevorzugtes Silikonöl ist Baysilone-Oel M 100 von Momentive Performance Materials.

Dimethicone kann auch als Mischung mit einem weiteren flüchtigen oder nichtflüchtigen Silikonöl vorhanden sein.

Die Behandlung einer Haarsträhne (2 g, 2 h gebleicht, 23 cm lang; Firma Kerling) mit 0,4 ml einer Spülung basierend auf einem tertiären Amin (Formelbeispiele 1 - 4), die einen Molenbruch des kationischen Tensids von x = 0,1 - 0,2 und eine interlamellar gebundene Wassermenge von > 50 % aufweist, führte zu einer von einem Expertenpanel (n=5) sichtbar verbesserten Produktreichhaltigkeit. Formulierungen mit einem Molenbruch von x > 0,3 zeigten eine deutlich geringere Pflegeleistung. Auch Zusammensetzungen mit geringerer gebundener Wassermenge hatten eine Verschlechterung der Pflegewirkung zur Folge.

Die folgenden Beispiele sollen den Gegenstand der vorliegenden Erfindung erläutern ohne ihn jedoch zu beschränken.

**Tabelle 1**: Beispiele

| Handelsname, Firma, Aktivgehalt | INCI | Bsp. 1 | Bsp. 2 | Bsp. 3 | Bsp. 4 |
|---|---|---|---|---|---|
| Solbrol M, Lanxess, 99.5% | Methylparaben | 0,25 | 0,25 | 0,25 | 0,25 |
| Propylparaben, Schütz & Co., 99.5% | Propylparaben | 0,10 | 0,10 | 0,10 | 0,10 |
| Nacol 16-95, Sasol, 95% | Cetyl Alcohol | 2,4 | 5,3 | 2,7 | 2,8 |
| Lanette 18, Cognis, 100% | Stearyl Alcohol | 4,6 | | 5,3 | 5,4 |
| Lanette 14, Cognis, 100% | Myristyl Alcohol | | 2,8 | | |
| Tego Amid S18, Evonik, 100% | Stearamidopropyl Dimethylamine | 2,5 | 1,4 | | |
| Amidet APA-22, Kao Chemicals, 100% | Behenamidopropyl Dimethylamine | | | 1,5 | |
| Varisoft PATC, Evonik, 60% | Palmitamidopropyltrimonium Chloride | | | | 2,3 |
| DC 5-7139, 65% Dimethicone | Dimethicone + Cocamidopropyl Betaine + C12-15 Pareth-3 + Guar Hydroxypropyltrimonium Chloride | 4,6 | 4,6 | 4,6 | 4,6 |
| Gamma Oryzanol, Jan Dekker, 98% | Oryzanol | 0,01 | 0,01 | 0,01 | 0,01 |
| Dehyton AB 30, Cognis, 31% | Coco Betaine | 0,85 | 0,85 | 0,85 | 0,85 |
| Sanal P, Akzo Nobel, 100% | Sodium Chloride | 0,01 | 0,01 | 0,01 | 0,01 |
| Lactic Acid, Biesterfeld, 90% | Lactic Acid | 0,80 | 0,80 | 0,80 | 0,80 |
| | Parfum | 0,70 | 0,70 | 0,70 | 0,70 |
| | Aqua | Ad. 100 | Ad. 100 | Ad. 100 | Ad. 100 |
| Gebundener Wasseranteil [%] | | 100 | 47,0 | 93,7 | 90,5 |
| Molenbruch | | 0,2 | 0,1 | 0,1 | 0,16 |

Beispiel 2 liegt außerhalb des Anspruchsbereichs.

Es hat sich gezeigt, dass diese erfindungsgemäßen Zubereitungen wirksam sind. Dazu wurden obige Zusammensetzungen auf ihre Anwendungseigenschaften untersucht. In einem Expertenpanel hat sich gezeigt, dass Zubereitungen mit einem hohen gebundenen Wasseranteil (Tabelle 2) zu einem hohen Gehalt der Formulierungen bei der Anwendung führen (Tabelle 3). Dabei sind insbesondere Zubereitungen mit einem Stoffmengenverhältnis der verwendeten Tenside von 0,1 - 0,3 sehr reichhaltig. Besonders bevorzugt werden außerdem Zubereitungen mit Cetyl- und Stearylalkohol und Stearamidopropyl Dimethylamine bzw. Behenamidopropyl Dimethylamine oder Palmitamidopropyltrimoniumchlorid.

**Tabelle 2:** Gebundener Wasseranteil [%]; kationische Tenside und Fettalkohole in Beispiel 1-4 laut Tabelle 1 variiert.

| Molenbruch | 0,1 | 0,2 | 0,3 | 0,4 | 0,5 |
|---|---|---|---|---|---|
| Stearamidopropyl Dimethylamine; Cetyl- und Stearylalkohol | 15,8 | 100,0 | 7,6 | 4,9 | 12,0 |
| Stearamidopropyl Dimethylamine; Myristyl- und Cetylalkohol | 47,0 | 20,0 | 7,0 | 1,3 | 16,0 |
| Behenamidopropyl Dimethylamine; Cetyl- und Stearylalkohol | 93,7 | 49,6 | 26,8 | 55,8 | 15,9 |
| Palmitamidopropyltrimmoniumchlorid; Cetyl- und Stearylalkohol | 90,5 | 80,0 | 4,7 | 0,0 | 0,1 |

**Tabelle 3:** Reichhaltigkeit bestimmt durch Expertenpanel (10 = am besten, 0=am schlechtesten)

| Molenbruch | 0,1 | 0,2 | 0,3 | 0,4 | 0,5 |
|---|---|---|---|---|---|
| Stearamidopropyl Dimethylamine; Cetyl- und Stearylalkohol | 8,0 | 8,3 | 5,5 | 4,5 | 5,5 |
| Stearamidopropyl Dimethylamine; Myristyl- und Cetylalkohol | 6,4 | 7,4 | 7,0 | 5,4 | 4,4 |
| Behenamidopropyl Dimethylamine; Cetyl- und Stearylalkohol |  | 8,6 |  |  |  |
| Palmitamidopropyltrimmoniumchlorid; Cetyl- und Stearylalkohol |  | 8,0 |  |  |  |

Sensorik-Protokoll für das Expertenpanel:

- Es werden 2 g einer für 2 Stunden gebleichten Haarsträhne verwendet.
- Die Haarsträhnen werden mindestens 15 min in Wasser eingeweicht.
- Die Hände werden mit Standardshampoo gewaschen.
- Die Haarsträhne wird entnommen und mit dem groben Kammsegment und danach mit dem feinen Kammsegment gekämmt.
- Die Haarsträhne wird unter fließendem Wasser nass gemacht und einmal mit leichtem Druck ausgestrichen.
- Eine Spritze wird mit 0,4 ml Produkt aufgezogen. Der Inhalt der Spritze wird auf die Haarsträhne aufgetragen.
- Die Hände werden mit Wasser aus einem Pumpspender befeuchtet.
- Das Produkt wird 60 s im angegebenen Takt (58 bpm) von oben nach unten verstrichen und danach 30 s einwirken gelassen. Anschließend wird das Produkt 50 s lang ausgespült. Die Haarsträhne wird mit dem groben Kammsegment und danach mit dem feinen Kammsegment gekämmt.
- Am Ende der Bewertung wird die Haarsträhne wieder nass gemacht und die ganze Prozedur wiederholt. Die Produkte werden nach einer Doppelbehandlung bewertet, um Unterschiede klarer darzustellen.

**Patentansprüche**

1. Kosmetische Zubereitung zur Behandlung keratinischer Fasern enthaltend kationische Tenside, wobei die kationischen Tenside Stearamidopropyl Dimethylamine, Behenamidopropyl Dimethylamine und/oder Palmitamidopropyltrimonium Chloride sind,
einen oder mehrere Fettalkohole mit einem Schmelzpunkt oberhalb 35°C und
Milchsäure
**dadurch gekennzeichnet, dass** der Anteil an interlamellar gebundenem Wasser wenigstens 50 %; gemessen mit $^1$H-NMR Diffusionsmessungen bei 25°C, beträgt und
dass das Stoffmengenverhältnis zwischen kationischen Tensiden zu der Summe der Stoffmengen an allen Tensiden und Fettverbindungen 10 bis 20 % beträgt.

2. Zubereitung nach Patentanspruch 1, **dadurch gekennzeichnet, dass** die Fettalkohole C14, C16, C18 Alkohole sind, bevorzugt eine Mischung aus Cetyl- und Stearylalkohol.

3. Zubereitung nach einem der vorangehenden Patentansprüche, **dadurch gekennzeichnet, dass** der Anteil an interlamellar gebundenem Wasser wenigstens 60 % beträgt.

4. Zubereitung nach einem der vorangehendenden Patentansprüche, **dadurch gekennzeichnet, dass** Fettalkohole in Mengen von 5,0 bis 8,5 Gew.-%, vorzugsweise 6,5 bis 8,0 Gew.-%, bezogen auf die gesamte Zusammensetzung, enthalten sind.

5. Verwendung von Zubereitungen nach einem der vorangehenden Patentansprüche zur Verbesserung der Pflege und Kämmbarkeit der Haare.

## Claims

1. Cosmetic preparation for treating keratin fibres, comprising cationic surfactants, wherein the cationic surfactants are stearamidopropyl dimethylamine, behenamidopropyl dimethylamine and/or palmitamidopropyltrimonium chloride,
one or more fatty alcohols having a melting point above 35°C, and
lactic acid,
**characterized in that** the proportion of interlamellarly bound water is at least 50%, measured by $^1$H NMR diffusion measurements at 25°C, and
**in that** the quantitative ratio of cationic surfactants to the sum of the quantities of all surfactants and fatty compounds is 10 to 20%.

2. Preparation according to Claim 1, **characterized in that** the fatty alcohols are C14, C16 or C18 alcohols, preferably a mixture of cetyl alcohol and stearyl alcohol.

3. Preparation according to one of the preceding claims, **characterized in that** the proportion of interlamellarly bound water is at least 60%.

4. Preparation according to one of the preceding claims, **characterized in that** fatty alcohols are present in amounts from 5.0 to 8.5 wt%, preferably 6.5 to 8.0 wt%, based on the total composition.

5. Use of preparations according to one of the preceding claims for improving the care and combability of the hair.

## Revendications

1. Préparation cosmétique pour le traitement de fibres kératiniques, contenant :

des tensioactifs cationiques, les tensioactifs cationiques étant la stéaramidopropyldiméthylamine, la béhénamidopropyldiméthylamine et/ou le chlorure de palmitamidopropyltrimonium,
un ou plusieurs alcools gras ayant un point de fusion supérieur à 35 °C et
de l'acide lactique,
**caractérisée en ce que** la proportion d'eau reliée sous forme inter-lamellaire est d'au moins 50 %, mesurée par des mesures de diffusion de RMN $^1$H à 25 °C, et
**en ce que** le rapport entre la quantité de matière des tensioactifs cationiques et la somme des quantités de matière de tous les tensioactifs et les composés gras est de 10 à 20 %.

2. Préparation selon la revendication 1, **caractérisée en ce que** les alcools gras sont des alcools en C14, C16, C18, de préférence un mélange d'alcool cétylique et stéarylique.

3. Préparation selon l'une quelconque des revendications précédentes, **caractérisée en ce que** la proportion d'eau reliée sous forme inter-lamellaire est d'au moins 60 %.

4. Préparation selon l'une quelconque des revendications précédentes, **caractérisée en ce que** des alcools gras sont contenus en quantités de 5,0 à 8,5 % en poids, de préférence de 6,5 à 8,0 % en poids, par rapport à l'ensemble de la composition.

5. Utilisation de préparations selon l'une quelconque des revendications précédentes pour l'amélioration du soin et du démêlage des cheveux.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009158441 A1
- WO 2009158442 A
- EP 1254653 B1
- EP 1131041 B1
- US 7601339 B2
- WO 2006137003 A2
- EP 2138154 A2

**In der Beschreibung aufgeführte Nicht-Patentliteratur**

- *Pharm. Ind.,* 1984, vol. 46, 758
- **STEJSKAL ; TANNER.** *J. Chem. Phys.,* 1965, vol. 42, 288
- **TANNER.** *J. Chem. Phys.,* 1970, vol. 52, 2523